# FASCICULE DE BREVET EUROPEEN

(11) **EP 0 969 795 B1**
(45) Date de publication et mention de la délivrance du brevet: **23.06.2004**
(21) Numéro de dépôt: 98945351.9
(22) Date de dépôt: 22.09.1998
(51) Int. Cl.: A61K 7/09, A61K 7/135, A61K 7/13

(54) **COMPOSITION OXYDANTE ET UTILISATIONS POUR LA TEINTURE, POUR LA DEFORMATION PERMANENTE OU POUR LA DECOLORATION DES FIBRES KERATINIQUES**
OXIDATIVE ZUSAMMENSETZUNG UND VERWENDUNG ZUM FÄRBEN, ZUR DAUERHAFTEN VERFORMUNG UND ZUM ENTFÄRBEN DER HAAREN
OXIDISING COMPOSITION AND USES FOR DYEING, PERMANENTLY SETTING OR BLEACHING KERATIN FIBRES

(30) Priorité: 03.10.1997 FR 9712358
(43) Date de publication de la demande: 12.01.2000
(73) Titulaire: L'OREAL, 75008 Paris (FR)
(72) Inventeur: DE LA METTRIE, Roland, F-78110 Le Vésinet (FR); COTTERET, Jean, F-78480 Verneuil sur Seine (FR); DE LABBEY, Arnaud, F-93600 Aulnay sous Bois (FR); MAUBRU, Mireille, F-78400 Chatou (FR)
(74) Mandataire: Miszputen, Laurent
(86) Numéro de dépôt international: PCT/FR1998/002027
(87) Numéro de publication internationale: WO 1999/017722

(56) Documents cités:
- EP-A- 0 310 675
- EP-A- 0 548 620
- EP-A- 0 548 621
- EP-A- 0 716 846
- EP-A- 0 795 313
- WO-A-94/00100
- DE-A- 19 547 991
- FR-A- 2 586 913

## Description

La présente invention a trait à une composition oxydante, destinée au traitement des fibres kératiniques comprenant au moins une enzyme de type oxydo-réductase à 2 électrons en présence d'au moins un donneur pour ladite enzyme et au moins une silicone aminée ainsi que ses utilisations pour la teinture, pour la déformation permanente ou pour la décoloration des fibres kératiniques en particulier des cheveux humains.

Il est connu de teindre les fibres kératiniques et en particulier les cheveux humains avec des compositions tinctoriales contenant des précurseurs de colorant d'oxydation, en particulier des paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant un précurseur de colorant d'oxydation en association avec des enzymes telles que la pyranose-oxydase, la glucose-oxydase ou bien l'uricase, en présence d'un donneur pour lesdites enzymes. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes, à la fois sur le plan de l'homogénéité de la couleur répartie le long de la fibre (« unisson »), sur le plan de la chromaticité (luminosité), de la puissance tinctoriale et de la résistance vis à vis des diverses agressions que peuvent subir les cheveux.

De même, la demande de brevet EP-A-0 716 846 divulgue une composition de teinture oxydante pour les cheveux contenant de l'uricase, de l'acide urique, une teinture d'oxydation et éventuellement un agent réducteur.
Des compositions similaires sont également décrites dans la demande de brevet DE 195 47 991, ainsi que l'utilisation de ces dernières dans la lutte contre les microorganismes, pour l'éclaircissement de la peau et/ou des cheveux et pour l'hydratation de la peau. Ces compositions peuvent également contenir de l'huile de silicone ou un copolyole de diméthicone.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses divers dérivés, la cystéamine et ses dérivés, l'acide thiolactique ou l'acide thioglycolique, leurs sels ainsi que leurs esters, notamment le thioglycolate de glycérol.

Concernant les compositions oxydantes nécessaires à la mise en oeuvre de l'étape de fixation, on fait le plus souvent appel, dans la pratique, à des compositions à base d'eau oxygénée, de bromate de sodium ou de persels comme le perborate de sodium, qui ont le désavantage d'être susceptibles d'abîmer les cheveux.

Le problème de la technique des permanentes connues à ce jour est que leur application sur les cheveux induit à la longue une altération de la qualité des cheveux. Les causes essentielles de cette altération de la qualité des cheveux sont une diminution de leurs propriétés cosmétiques, telles que leur brillance, le toucher et une dégradation de leurs propriétés mécaniques, plus particulièrement une dégradation de leur résistance mécanique due à un gonflement des fibres kératiniques lors du rinçage entre l'étape de réduction et l'étape d'oxydation qui peut également se traduire par une augmentation de leur porosité. Les cheveux sont affaiblis et peuvent devenir cassants lors de traitements ultérieurs comme des brushings.

On retrouve le même problème d'altération de la fibre kératinique lors des procédés de décoloration des cheveux.

On sait que la déformation permanente ou la décoloration des fibres kératiniques peut également être réalisée dans des conditions plus douces à l'aide de systèmes oxydants différents du peroxyde d'hydrogène tels que des systèmes enzymatiques. Ainsi il a déjà été proposé des procédés de déformation permanente ou décoloration des fibres kératiniques, notamment dans la demande de brevet EP-A-0 310 675, avec des compositions comprenant une enzyme telle que la pyranose-oxydase, la glucose-oxydase ou bien l'uricase, en présence d'un donneur pour ladite enzyme. Ces formulations oxydantes, bien qu'étant mises en oeuvre dans des conditions n'entraînant pas une dégradation des fibres kératiniques comparable à celle engendrée par les procédés classiques de permanente ou de décoloration, conduisent à des résultats encore insuffisants quant à la tenue de la frisure dans le temps, quant à la compatibilité des cheveux permanentés ou décolorés avec les traitements ultérieurs, quant à la réduction de la dégradation des propriétés mécaniques des cheveux permanentés notamment la réduction de la porosité des cheveux, quant aux propriétés cosmétiques telles que le toucher ou bien encore sur le plan de l'uniformité de la décoloration le long des fibres kératiniques.

La présente invention a pour but de résoudre les problèmes évoqués ci-dessus.

La Demanderesse a découvert de façon surprenante de nouvelles compositions contenant au moins comme système oxydant une enzyme de type oxydo-réductase à 2 électrons en présence d'au moins un donneur pour ladite enzyme et au moins une silicone aminée, qui peuvent constituer en présence de précurseurs de colorant d'oxydation (bases d'oxydation) et d'éventuellement de coupleurs, des formulations de teinture prêtes à l'emploi, conduisant à des colorations plus homogènes, plus puissantes et plus chromatiques sans engendrer de dégradation significative, peu sélectives et résistant bien aux diverses agressions que peuvent subir les cheveux.

La Demanderesse a découvert également de façon inattendue que l'utilisation dans un procédé de déformation permanente des fibres kératiniques d'une composition oxydante contenant au moins comme système oxydant une enzyme de type oxydo-réductase à 2 électrons en présence d'au moins un donneur pour ladite enzyme et au moins une silicone aminée, permettait de résoudre les problèmes techniques évoqués ci-dessus. En particulier, ce type de composition oxydante permet d'améliorer la tenue à la frisure obtenue dans le temps, de réduire sensiblement la porosité des cheveux permanentes, d'améliorer la compatibilité des cheveux permanentés aux traitements ultérieurs.

La Demanderesse a découvert également de façon surprenante que l'utilisation, dans un procédé de décoloration des fibres kératiniques, d'une composition oxydante contenant au moins comme système oxydant une enzyme de type oxydo-réductase à 2 électrons en présence d'au moins un donneur pour ladite enzyme et au moins une silicone aminée, permettait de résoudre les problèmes techniques évoqués ci-dessus notamment d'améliorer la compatibilité des cheveux décolorés aux traitements ultérieurs. Ce type de composition oxydante permet d'obtenir un effet décolorant plus uniforme sur les cheveux et d'améliorer les propriétés cosmétiques, comme le toucher.

Ces découvertes sont à la base de la présente invention.

La présente invention a donc pour premier objet une composition cosmétique et/ou dermatologique destinée au traitement des fibres kératiniques, en particulier des fibres kératiniques humaines et plus particulièrement des cheveux humains, comprenant dans un support approprié pour les fibres kératiniques :
(a) au moins une enzyme de type oxydo-réductase à 2 électrons en présence d'au moins un donneur pour ladite enzyme
(b) au moins une silicone aminée.

La ou les oxydo-réductases à 2 électrons utilisées dans les compositions oxydantes conformes à l'invention peuvent notamment être choisies parmi les pyranose oxydases, les glucose oxydases, les glycérol oxydases, les lactate oxydases, les pyruvate oxydases, et les uricases.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition, et encore plus préférentiellement de 0,1 à 5 % en poids environ de ce poids.

Selon l'invention, on entend par donneur, les différents substrats également nécessaires au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose; à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; et enfin à titre de donneur pour les uricases, on peut citer l'acide urique et ses sels.

Le ou les donneurs (ou substrats) utilisés conformément à l'invention représentent de préférence de 0,01 à 20 % en poids environ du poids total de la composition conforme à l'invention et encore plus préférentiellement de 0,1 à 5 % en environ de ce poids.

Selon l'invention, on désigne par silicone aminée toute silicone comportant au moins une amine primaire, secondaire, tertiaire ou un groupement ammonium quaternaire. On peut ainsi citer :
**(a)** les polysiloxanes dénommés dans le dictionnaire CTFA "amodiméthicone" et répondant à la formule (I): 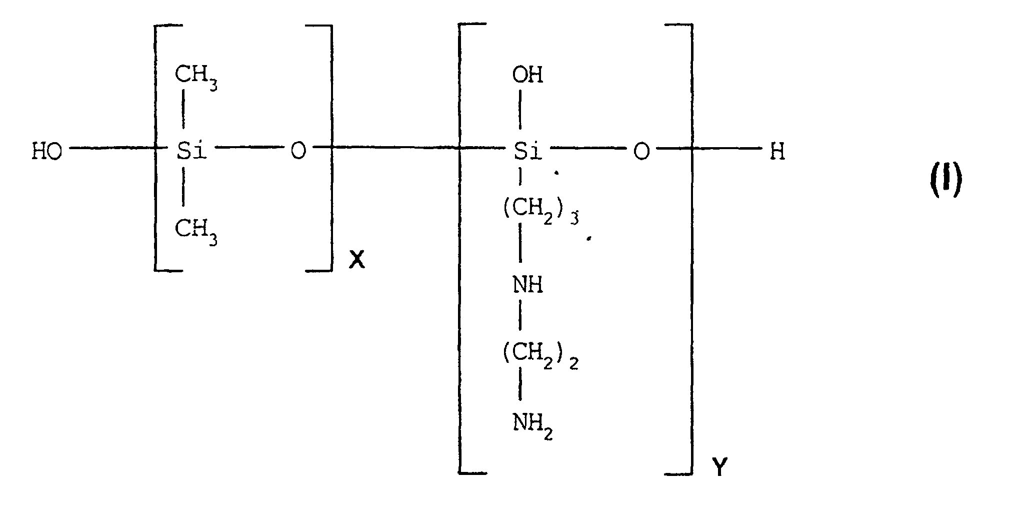 dans laquelle x et y sont des nombres entiers dépendant du poids moléculaire, généralement tels que ledit poids moléculaire en nombre est compris entre 5 000 et 500 000 environ ;
**(b)** les polymères cationiques siliconés répondant à la formule :

   RₐG₃₋ₐ-Si(OSiG₂)ₙ-(OSiG_{b}R_{2-b})ₘ-O-SiG₃₋ₐ-Rₐ (II)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R est un radical monovalent de formule -C_{q}H_{2q}L dans laquelle q est un nombre de 2 à 8 et L est un groupement aminé éventuellement quaternisé choisi parmi les groupements :

      -N(R')-Ch₂-CH₂-N(R')₂

      -N(R')₂

      -N⊕(R')₃ A⁻

      -N⊕(R')₂ (H) A⁻

      -N(R') (H)₂ A⁻

      -N(R')-CH₂-CH₂-N⊕(R') (H)₂ A⁻ ;
   dans lesquels R' peut désigner hydrogène, phényle, benzyle, ou un radical hydrocarboné saturé monovalent, par exemple un radical alkyle ayant de 1 à 20 atomes de carbone et A représente un ion halogénure tel que par exemple fluorure, chlorure, bromure ou iodure.
   Un produit correspondant à cette définition est le polymère dénommé "triméthylsilylamodiméthicone", répondant à la formule (III) suivante: 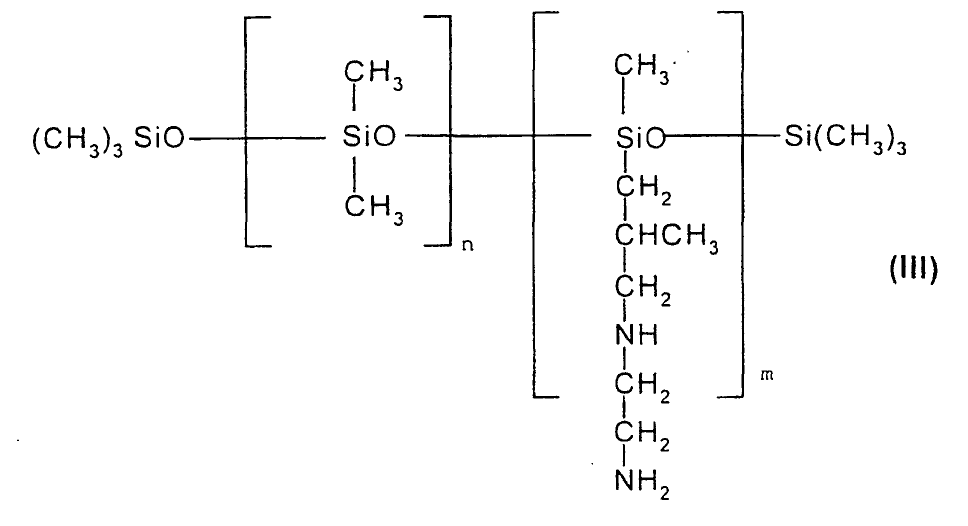 dans laquelle n et m ont les significations données ci-dessus pour la formule (II). De tels polymères sont décrits par exemple dans la demande de brevet EP-A-95238.
**(c)** les polymères cationiques siliconés répondant à la formule : 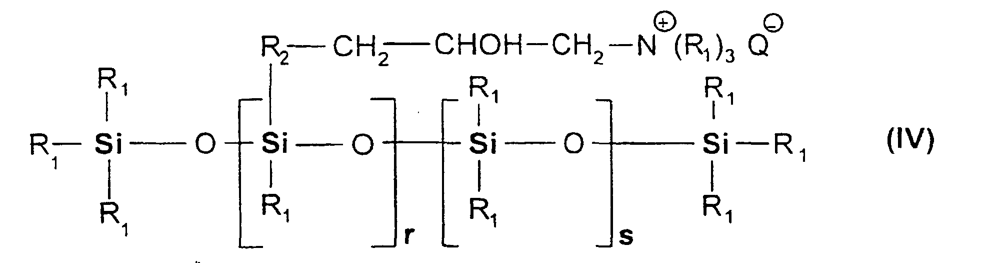 dans laquelle
   R, représente un radical hydrocarboné monovalent ayant de 1 à 18 atomes de carbone, et en particulier un radical alkyle en C₁-C₁₈, ou alcényle en C₂-C₁₈, par exemple méthyle ;
   R₂ représente un radical hydrocarboné divalent, notamment un radical alkylène en C₁-C₁₈ ou un radical alkylèneoxy divalent en C₁-C₁₈, par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

De tels polymères sont décrits plus particulièrement dans le brevet US-4 185 087.

Un polymère entrant dans cette classe est le polymère vendu par la Société Union Carbide sous la dénomination "Ucar Silicone ALE 56.

Lorsque ces polymères siliconés sont mis en oeuvre, une forme de réalisation particulièrement intéressante est leur utilisation conjointe avec des agents de surface cationiques et/ou non ioniques.
On peut utiliser par exemple le produit vendu sous la dénomination "Emulsion Cationique DC 929" par la Société Dow Corning qui comprend, outre l'amodiméthicone, un agent de surface cationique comprenant un mélange de produits répondant à la formule : 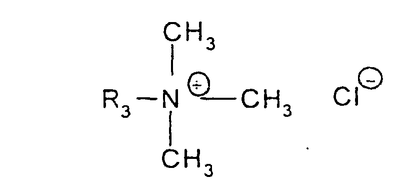 dans laquelle R₃ désigne des radicaux alcényle et/ou alcoyle ayant de 14 à 22 atomes de carbone, dérivés des acides gras du suif, et connu sous la dénomination CTFA "tallowtrimonium chloride", en association avec un agent de surface non ionique de formule : C₉H₁₉-C₆H₄-(OC₂H₄)₁₀-OH , connu sous la dénomination "Nonoxynol 10".

On peut également utiliser par exemple le produit vendu sous la dénomination "Emulsion Cationique DC 939" par la Société Dow Corning qui comprend, outre l'amodiméthicone, un agent de surface cationique qui est le chlorure de triméthyl cétylammonium, en association avec un agent de surface non ionique de formule C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association, le triméthylsilylamodiméthicone de formule (III) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

Les compositions conformes à l'invention renferment les silicones aminées définies ci-dessus à des teneurs pondérales qui peuvent être comprises entre 0,05 % et 10 %, de préférence entre 0,1 % et 5 % et encore plus préférentiellement entre 0,2 % et 3%, par rapport au poids total de la composition.

La présente invention a également pour objet une composition prête à l'emploi, pour la teinture d'oxydation des fibres kératiniques et en particulier des fibres kératiniques humaines telles que les cheveux, du type comprenant, dans un milieu approprié pour la teinture, au moins une base d'oxydation et, le cas échéant, un ou plusieurs coupleurs, qui est caractérisée par le fait qu'elle contient :
(a) au moins une enzyme de type oxydo-réductase à 2 électrons en présence d'au moins un donneur pour ladite enzyme
(b) au moins une silicone aminée.

La nature de la ou des bases d'oxydation utilisées dans la composition tinctoriale prête à l'emploi n'est pas critique. Elles peuvent notamment être choisies parmi les paraphénylènediamines, les bases doubles, les para-aminophénols, les ortho aminophénols et les bases d'oxydation hétérocycliques, ainsi que les sels d'addition de ces composés avec un acide.

Parmi les paraphénylènediamines utilisables à titre de base d'oxydation dans les composition tinctoriales conformes à l'invention, on peut notamment citer les composés de formule (V) suivante et leurs sels d'addition avec un acide : 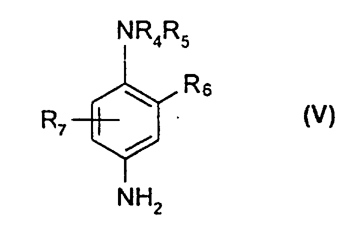 dans laquelle :
- R₄ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₅ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₆ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₇ représente un atome d'hydrogène, d'halogène ou un radical alkyle en C₁-C₄.

Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (V) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine; la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (V) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine. la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (VI) suivante, et leurs sels d'addition avec un acide : 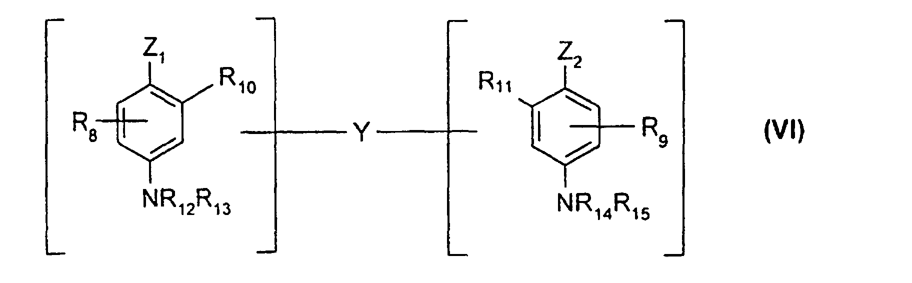 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₈ et R₉ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₀, R₁₁, R₁₂, R₁₃ R₁₄ et R₁₅, identiques ou différents, représentent un atome d'hydrogène, un bras de liaison Y ou un radical alkyle en C₁-C₄ ;
étant entendu que les composés de formule (VI) ne comportent qu'un seul bras de liaison Y par molécule.

Parmi les groupements azotés de la formule (VI) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (VI) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (VI), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (VII) suivante, et leurs sels d'addition avec un acide : 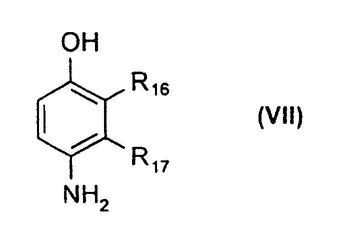 dans laquelle :
- R₁₆ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₇ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₆ ou R₁₇ représente un atome d'hydrogène.

Parmi les para-aminophénols de formule (VII) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer ies dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-((3-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 ou la demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (VIII) suivante, leurs sels d'addition avec un acide ou avec une base et leurs formes tautomères; lorsqu'il existe un équilibre tautomérique : 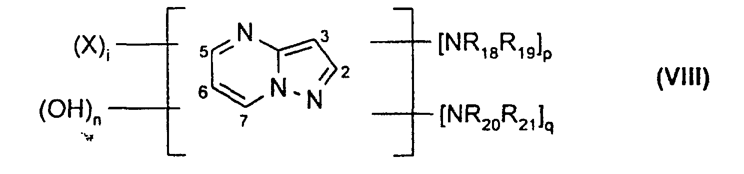 dans laquelle:
- R₁₈, R₁₉, R₂₀ et R₂₁, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C,-C₄)alkyl- ou di-[hydroxy(C₁-C₄) alkyl)-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C₄)alkyl ou di-[hydroxy(C₁-C₄ )alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;

- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₈R₁₉ et NR₂₀R₂₁ occupent les positions (2,3) ; (5,6) ; (6,7) ; (3,5) ou (3,7) ;
- lorsque p + q est égal à 1 alors n vaut 1 et le groupe NR₁₈R₁₉ (ou NR₂₀R₂₁) et le groupe OH occupent les positions (2,3) ; (5,6) ; (6,7) ; (3,5) ou (3,7) ;

Lorsque les pyrazolo-[1,5-a]-pyrimidines de formule (VIII) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

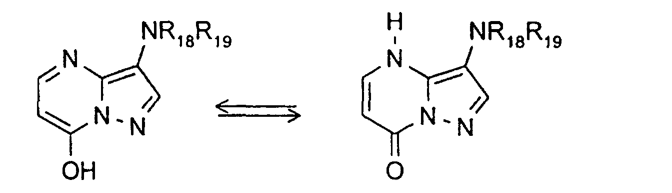

Parmi les pyrazolo-[1,5-a]-pyrimidines de formule (VIII) ci-dessus on peut notamment citer :
- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- ie 3-amino pyrazoio-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (VIII) ci-dessus peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-Al, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (VIII) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Almera, J. Elguero, J. Heterocyclic Chem., 11(3), 423, 1974.
- K. Saito, I. Hori, M. Higarashi, H. Midorikawa, Bull. Chem. Soc. Japan, 47(2), 476, 1974.

La ou les bases d'oxydation conformes à l'invention représentent de préférence de 0,0005 à 12 % en poids environ du poids total de la composition tinctoriale prête à l'emploi, et encore plus préférentiellement de 0,005 à 6 % en poids environ de ce poids.

Les coupleurs utilisables sont ceux classiquement utilisés dans les compositions de teinture d'oxydation, c'est-à-dire des métaphénylènediamines, des métaaminophénols et des métadiphénols, les dérivés mono- ou poly-hydroxylés du naphtatène, le sésamol et ses dérivés et des composés hétérocycliques tels que par exemple les dérivés indoliques, les dérivés indoliniques, les dérivés de benzimidazole, les dérivés de benzomorpholine, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoiiques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, et leurs sels d'addition avec un acide.

Lorsqu'ils sont présents ces coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

La composition tinctoriale de l'invention peut encore contenir, en plus des précurseurs de colorants d'oxydation définis ci-dessus et des éventuels coupleurs associés, des colorants directs pour enrichir les nuances en reflets. Ces colorants directs peuvent notamment alors être choisis parmi les colorants nitrés, azoïques ou anthraquinoniques.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins une base d'oxydation et éventuellement au moins un coupleur tel que défini précédemment et, d'autre part, une composition (B) renfermant, dans un milieu approprié pour la teinture, au moins une enzyme de type oxydo-réductase à 2 électrons en présence d'au moins un donneur pour ladite enzyme et au moins une silicone aminée, puis à procéder à leur mélange au moment de l'emploi avant d'appliquer ce mélange sur les fibres kératiniques.

Selon une autre forme de réalisation particulière de l'invention, la silicone aminée est incorporée dans la composition (A).

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

La présente invention a également pour objet un nouveau procédé de traitement des matières kératiniques, en particulier des cheveux, en vue d'obtenir une déformation permanente de ces dernières, en particulier sous la forme de cheveux permanentés, ce procédé comprenant les étapes suivantes : (i) on applique sur la matière kératinique à traiter une composition réductrice, la matière kératinique étant mise sous tension mécanique avant, pendant, ou après ladite application, (ii) on rince éventuellement la matière kératinique, (iii) on applique sur la matière kératinique éventuellement rincée une composition oxydante telle que définie ci-dessus, (iv) on rince éventuellement à nouveau la matière kératinique.

La première étape (i) de ce procédé consiste à appliquer sur les cheveux une composition réductrice. Cette application se fait mèche par mèche ou globalement.

La composition réductrice comprend par exemple au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Les cheveux peuvent également être mis en forme sans l'aide de moyens extérieurs, simplement avec les doigts.

Avant de procéder à l'étape (ii) suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape, facultative, du procédé (étape (ii)), les cheveux imprégnés de la composition réductrice sont donc ensuite rincés soigneusement par une composition aqueuse.

Puis, dans une troisième étape (étape (iii)), on applique sur les cheveux ainsi rincés la composition oxydante de l'invention, dans le but de fixer la nouvelle forme imposée aux cheveux.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention (étape (iv)), étape facultative également, les cheveux imprégnés de la composition oxydante sont rincés soigneusement, généralement à l'eau.

On obtient finalement une chevelure facile à démêler, douce. Les cheveux sont ondulés.

La composition oxydante selon l'invention peut également être utilisée dans un procédé de décoloration des fibres kératiniques, et en particulier des cheveux.

Le procédé de décoloration selon l'invention comprend une étape d'application sur les fibres kératiniques d'une composition oxydante selon l'invention en présence ou non d'un oxydant auxiliaire. Classiquement, une deuxième étape du procédé de décoloration selon l'invention est une étape de rinçage des fibres kératiniques.

Le milieu approprié pour les fibres kératiniques (ou support) des compositions tinctoriales prêtes à l'emploi et des compositions oxydantes utilisées pour la déformation permanente ou la décoloration des fibres kératiniques conformes à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH des compositions tinctoriales prêtes à l'emploi et des compositions oxydantes utilisées pour la déformation permanente ou la décoloration des fibres kératiniques conformes à l'invention est choisi de telle manière que l'activité enzymatique de l'oxydo-réductase à 2 électrons ne soit pas altérée. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (IX) suivante 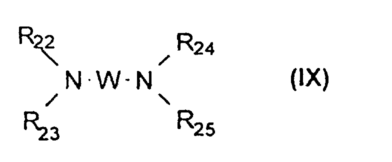 dans laquelle W est un reste propylène éventuellement substitué par un groupement hydroxyle ou un radical alkyle en C₁-C₄ ; R₂₂, R₂₃, R₂₄ et R₂₅, identiques ou différents, représentent un atome d'hydrogène, un radical alkyle en C₁-C₄ ou hydroxyalkyle en C₁-C₄.

Les compositions tinctoriales prêtes à l'emploi et les compositions oxydantes pour la déformation permanente ou la décoloration des fibres kératiniques conformes à l'invention peuvent également renfermer divers adjuvants utilisés classiquement dans les compositions pour la teinture, la déformation permanente ou la décoloration des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes des oxydo-réductases à 2 électrons utilisées conformément à l'invention telles que par exemples des peroxydases, des agents de pénétration, des agents séquestrants, des parfums, des tampons, des agents dispersants, des agents de conditionnement, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement aux compositions conformes à l'invention ne soient pas, ou substantiellement pas, altérées par la ou les adjonctions envisagées.

Les compositions tinctoriales prêtes à l'emploi et les compositions oxydantes utilisées pour la déformation permanente ou la décoloration des fibres kératiniques conformes à l'invention, peuvent se présenter sous des formes diverses, telles que sous forme de liquides, de crèmes, de gels, éventuellement pressurisés, ou sous toute autre forme appropriée pour réaliser une teinture, une déformation permanente ou une décoloration des fibres kératiniques, et notamment des cheveux humains.

Dans le cas d'une composition tinctoriale prête à l'emploi, le ou les colorants d'oxydation et la ou les oxydo-réductases à 2 électrons sont présents au sein de ladite composition qui doit être exempte d'oxygène gazeux, de manière à éviter toute oxydation prématurée du ou des colorants d'oxydation.

Des exemples concrets illustrant l'invention vont maintenant être donnés.

Dans ce qui suit ou ce qui précède, sauf mention contraire, les pourcentages sont exprimés en poids.

Les exemples suivants illustrent l'invention sans présenter un caractère limitatif.

### Exemple 1 de composition de teinture :

On a préparé la composition tinctoriale prête à l'emploi suivante (teneurs en grammes) :
- Uricase d'Arthrobacter globiformis à 20 Unités Internationales (U.I.) / mg, commercialisée par la société Sigma 1,5 g
- Acide urique 1,5 g
- Paraphénylènediamine 0,324 g
- Résorcine 0,33 g
- Polydiméthyl siloxane à groupements amino éthylamino propyle à 35 % dans l'eau, vendue sous le nom de DOW CORNING 939 EMULSION 1,2 g Eau déminéralisée qsp 100 g

La composition tinctoriale prête à l'emploi décrite ci-dessus a été appliquée sur des mèches de cheveux gris naturels à 90 % de blancs pendant 30 minutes. Les cheveux ont ensuite été rincés, lavés avec un shampooing standard, puis séchés.

On a obtenu des mèches de cheveux teintes en blond foncé mat.

### Exemple 2: Composition oxydante pour permanente ou décoloration

- Uricase d'Arthrobacter globiformis à 20 Unités Internationales (U.I.) / mg, commercialisée par la société Sigma 1,8 g
- Acide urique 1,65 g
- Ethanol 20,0 g
- Alkyl (C₈-C₁₀) polyglucoside en solution aqueuse à 60 % de matière active (M.A.) vendu sous la dénomination ORAMIX CG110 par la société SEPPIC 8,0 g
- Mélange de polydiméthylsiloxane à groupements aminoéthyl aminoisobutyle/polydiméthylsiloxane vendu sous le nom commercial DOW CORNING Q2 8220 FLUID 1,0 g
- 2-méthyl-2-amino propanol-1 qs pH 9,5
- Eau déminéralisée qsp 100 g

## Revendications

1. Composition cosmétique et/ou dermatologique destinée au traitement des fibres kératiniques, en particulier des fibres kératiniques humaines et plus particulièrement les cheveux humains, comprenant dans un support approprié pour les fibres kératiniques :
(a) au moins une enzyme de type oxydo-réductase à 2 électrons en présence d'au moins un donneur pour ladite enzyme
(b) au moins une silicone aminée.

2. Composition selon la revendication 1, **caractérisée par le fait que** l'oxydo-réductases à 2 électrons est choisie parmi les uricases d'origine animale, microbiologique ou biotechnologique.

3. Composition selon la revendication 1 ou 2, **caractérisée par le fait que** la ou les oxydo-réductases à 2 électrons représentent de 0,01 à 20 % en poids du poids total de la composition.

4. Composition selon la revendication 3, **caractérisée par le fait que** la ou les oxydo-réductases à 2 électrons représentent de 0,1 à 5 % en poids du poids total de la composition.

5. Composition selon la revendication 2, **caractérisée par le fait que** le donneur (ou substrat) pour ladite oxydo-réductase à 2 est choisi parmi l'acide urique et ses sels.

6. Composition selon l'une quelconque des revendications précédentes, **caractérisée par le fait que** le ou les donneurs représentent de 0,01 à 20 % en poids du poids total de la composition.

7. Composition selon la revendication 6, **caractérisée par le fait que** le ou les donneurs représentent de 0,1 à 5 % en poids du poids total de la composition.

8. Composition selon l'une quelconque des revendications 1 à 7, **caractérisée par le fait que** la silicone aminée est choisie parmi :
**(a)** les polysiloxanes répondant à la formule : 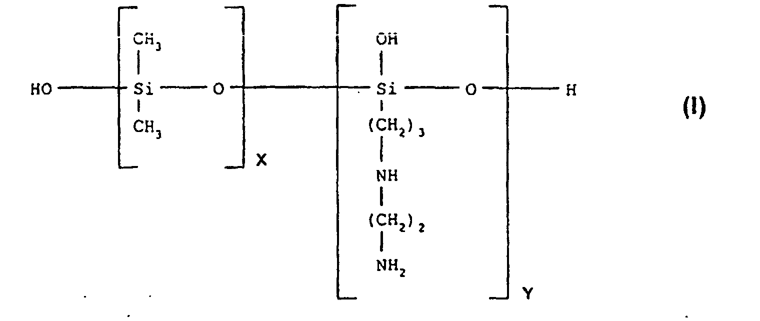 dans laquelle x et y sont des nombres entiers dépendant du poids moléculaire, généralement tels que ledit poids moléculaire en nombre est compris entre 5 000 et 500 000 environ ;
**(b)** les polymères cationiques siliconés répondant à la formule :
RₐG₃₋ₐ-Si(OSiG₂)ₙ-(OSiG_{b}R_{2.b})ₘ-O-SiG₃₋ₐ-Rₐ **(II)**
dans laquelle:
G est un atome d'hydrogène, ou un groupement phényle, OH, ou alkyle en C₁-C₈, par exemple méthyle,
a désigne le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R est un radical monovalent de formule -C_{q}H_{2q}L dans laquelle q est un nombre de 2 à 8 et L est un groupement aminé éventuellement quaternisé choisi parmi les groupements :
-N(R')-CH₂ CH₂-N(R')₂
-N(R')₂
-N^{⊕}(R')₃ A⁻
-N^{⊕}(R')₂ (H) A⁻
-N^{⊕}(R') (H)₂ A⁻
-N(R')-CH₂-CH₂-N⊕ (R') (H)₂ A- ;
dans lesquels, R' peut désigner hydrogène, phényle, benzyle, ou un radical hydrocarboné saturé monovalent, par exemple un radical alkyle ayant de 1 à 20 atomes de carbone et A représente un ion halogénure tel que par exemple fluorure, chlorure, bromure ou iodure.
**(c)** les polymères cationiques siliconés répondant à la formule : 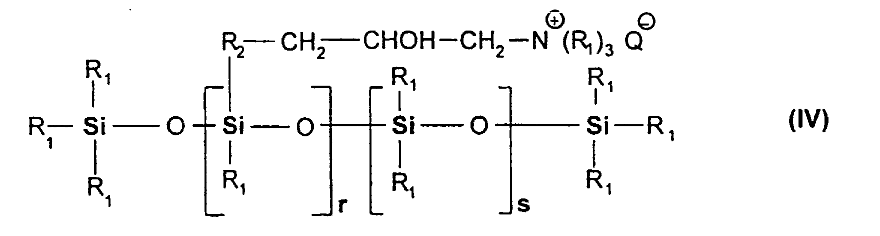 dans laquelle,
R₁ représente un radical hydrocarboné monovalent ayant de 1 à 18 atomes de carbone, et en particulier un radical alkyle en C₁-C_{18,} ou alcényle en C₂-C_{18,} par exemple méthyle ;
R₂ représente un radical hydrocarboné divalent, notamment un radical alkylène en C₁-C₁₈ ou un radical alkylèneoxy divalent en C₁-C₁₈, par exemple en C₁-C₈ ;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

9. Composition selon l'une quelconque des revendications 1 à 8, **caractérisée par le fait que** la concentration en silicone aminée varie de 0,05 % à 10 % en poids par rapport au poids total de la composition, et de préférence entre 0,1 et 5 %.

10. Composition selon l'une quelconque des revendications 1 à 9, prête à l'emploi, pour la teinture d'oxydation des fibres kératiniques et en particulier des fibres kératiniques humaines telles que les cheveux, du type comprenant, dans un milieu approprié pour les fibres kératiniques, en outre au moins base d'oxydation et, éventuellement un ou plusieurs coupleurs.

11. Composition selon la revendication 10, **caractérisée par le fait que** les bases d'oxydation sont choisis parmi les paraphénylènediamines, les bases doubles, les ortho- ou para- aminophénols, et les bases hétérocycliques, ainsi que les sels d'addition de ces composés avec un acide.

12. Composition selon la revendication 10 ou 11, **caractérisée par le fait que** les bases d'oxydation sont présentes dans des concentrations allant de 0,0005 à 12% en poids par rapport au poids total de la composition.

13. Composition selon la revendication 10, **caractérisée par le fait que** les coupleurs sont choisis parmi les métaphénytènediamines, les métaaminophénols, les métadiphénols, les coupleurs hétérocycliques, et les sels d'addition de ces composés avec un acide.

14. Composition selon la revendication 10 ou 13, **caractérisée par le fait que** les coupleurs sont présents dans des concentrations allant de 0,0001 à 10% en poids par rapport au poids total de la composition.

15. Composition selon la revendication 11 ou 13, **caractérisée par** ie fait que les sels d'addition avec un acide des bases d'oxydation et des coupleurs sont choisis parmi les chlorhydrates, les bromhydrates, les sulfates, les tartrates, les lactates et les acétates.

16. Composition selon l'une quelconque des revendications 10 à 15, **caractérisée par le fait qu'**elle contient en outre des colorants directs.

17. Composition selon l'une quelconque des revendications 1 à 16, **caractérisée par le fait que** le milieu approprié pour les fibres kératiniques (ou support) est constitué par de l'eau ou par un mélange d'eau et d'au moins un solvant organique.

18. Composition selon la revendication 17, **caractérisée par le fait que** les solvants organiques peuvent être présents dans des proportions de préférence allant 1 à 40 % en poids par rapport au poids total de la composition, et encore plus préférentiellement allant de 5 à 30 % en poids.

19. Composition selon l'une quelconque des revendications 1 à 18, **caractérisée par le fait que** le pH varie de 5 à 11, et de préférence de 6,5 à 10.

20. Composition selon l'une quelconque des revendications 1 à 19, **caractérisée par le fait qu'**elle contient en plus au moins un adjuvant cosmétique utilisé classiquement dans les compositions pour la teinture, la déformation permanente ou la décoloration des cheveux, choisi dans le groupe constitué par des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes des oxydo-réductases à 2 électrons, des agents de pénétration, des agents séquestrants, des parfums, des tampons, des agents dispersants, des agents de conditionnement, des agents filmogènes, des agents conservateurs, des agents opacifiants.

21. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications 10 à 20, pendant un temps suffisant pour développer la coloration désirée.

22. Procédé selon la revendication 21, **caractérisé par le fait qu'**il comporte une étape préliminaire consistant à stocker sous forme séparée, d'une part, une composition (A) comprenant, dans un milieu approprié pour la teinture, au moins une base d'oxydation et éventuellement au moins un coupleur tels que définis dans l'une quelconque des revendications précédentes et d'autre part, une composition (B) renfermant, dans un milieu approprié pour les fibres kératiniques, au moins une enzyme de type oxydo-réductase à 2 électrons en présence d'au moins un donneur pour ladite enzyme telle que défini dans l'une quelconque des revendications précédentes puis à procéder à leur mélange au moment de l'emploi avant d'appliquer ce mélange sur les fibres kératiniques ; la composition (A) ou la composition (B) contenant la silicone aminée telle que définie dans les revendications précédentes.

23. Dispositif à plusieurs compartiments ou "kit" de teinture, **caractérisé par le fait qu'**il comporte un premier compartiment renfermant la composition (A) telle que définie dans la revendication 22 et un second compartiment renfermant la composition (B) telle que définie dans la revendication 22.

24. Procédé de traitement des fibres kératiniques, en particulier des cheveux, en vue d'obtenir une déformation permanente de ces dernières, en particulier sous la forme de cheveux permanentés, comprenant les étapes suivantes : (i) on applique sur les fibres kératiniques à traiter une composition réductrice, la matière kératinique étant mise sous tension mécanique avant, pendant, ou après ladite application, (ii) on rince éventuellement la matière kératinique, (iii) on applique sur la matière kératinique éventuellement rincée une composition oxydante telle que définie dans l'une quelconque des revendications1 à 9, 17 à 20, (iv) on rince éventuellement à nouveau la matière kératinique.

25. Procédé de traitement des fibres kératiniques, en particulier des cheveux, en vue d'obtenir une décoloration de ces dernières comprenant l'application d'une composition oxydante telle que définie dans l'une quelconque des revendications 1 à 9, 17 à 20 contenant éventuellement un agent oxydant auxiliaire et une deuxième étape de rinçage des fibres kératiniques.

## Patentansprüche

1. Kosmetische und/oder dermatologische Zusammensetzung zur Behandlung von Keratinfasern, insbesondere menschlichen Keratinfasern und besonders menschlichen Haaren, die in einem für Keratinfasern geeigneten Träger enthält:
(a) mindestens ein Enzym vom Typ der Oxidoreduktasen, die 2 Elektronen übertragen, in Gegenwart mindestens eines Donors für das Enzym, und
(b) mindestens ein aminiertes Silicon.

2. Zusammensetzung nach Anspruch 1, **dadurch gekennzeichnet, dass** die Oxidoreduktase(n) (2 Elektronen) unter den Uricasen tierischer, mikrobiologischer oder biotechnologischer Herkunft ausgewählt sind.

3. Zusammensetzung nach Anspruch 1 oder 2, **dadurch gekennzeichnet, dass** die Oxidoreduktase(n) (2 Elektronen) 0,01 bis 20 Gew.-% des Gesamtgewichts der Zusammensetzung ausmachen.

4. Zusammensetzung nach Anspruch 3, **dadurch gekennzeichnet, dass** die Oxidoreduktase(n) (2 Elektronen) 0,1 bis 5 Gew.-% des Gesamtgewichts der Zusammensetzung ausmachen.

5. Zusammensetzung nach Anspruch 2, **dadurch gekennzeichnet, dass** der Donor (oder das Substrat) für die Oxidoreduktase (2 Elektronen) unter Harnsäure und ihren Salzen ausgewählt ist.

6. Zusammensetzung nach einem der vorhergehenden Ansprüche, **dadurch gekennzeichnet, dass** der Donor oder die Donoren 0,01 bis 20 Gew.-% des Gesamtgewichts Zusammensetzung ausmachen.

7. Zusammensetzung nach Anspruch 6, **dadurch gekennzeichnet, dass** der Donor oder die Donoren 0,1 bis 5 Gew.-% des Gesamtgewichts der Zusammensetzung ausmachen.

8. Zusammensetzung nach einem der Ansprüche 1 bis 7, **dadurch gekennzeichnet, dass** das aminierte Silicon unter den folgenden Verbindungen ausgewählt ist:
a) den Polysiloxanen der folgenden Formel: 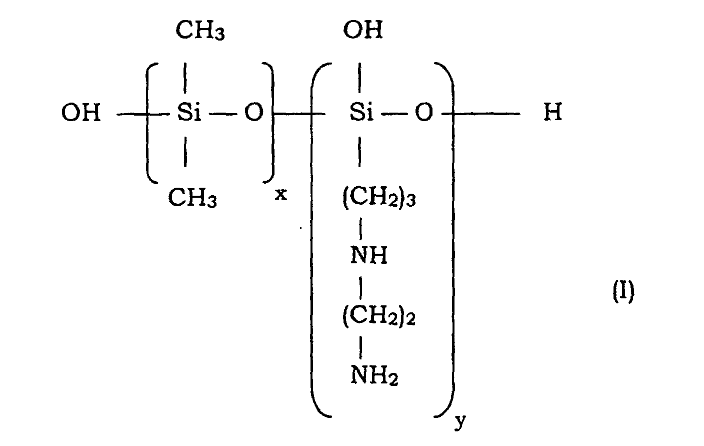 worin x und y ganze Zahlen bedeuten, die von der Molmasse abhängen und im Allgemeinen so ausgewählt sind, dass die zahlenmittlere Molmasse im Bereich von etwa 5 000 bis 500 000 liegt.
b) den kationischen siliconierten Polymeren der folgenden Formel:
RₐG₃₋ₐ-Si(OSiG₂)ₙ-(OSiG_{b}R_{2-b})ₘ-O-SiG₃₋ₐ-Rₐ (II),
worin bedeuten:
· die Gruppe G Wasserstoff, Phenyl, OH oder C₁₋₈-Alkyl, beispielsweise Methyl,
· a 0 oder eine ganze Zahl von 1 bis 3 und insbesondere 0,
· b 0 oder 1 und insbesondere 1,
· m und n Zahlen, die so ausgewählt sind, dass die Summe (n + m) insbesondere im Bereich von 1 bis 2000 und besonders 50 bis 150 liegen kann, wobei n eine Zahl von 0 bis 1999 und insbesondere 49 bis 149 und m eine Zahl von 1 bis 2000 und insbesondere 1 bis 10 bedeuten kann,
· die Gruppe R eine einwertige Gruppe der Formel -C_{q}H_{2q}L, worin q eine Zahl von 2 bis 8 ist und L eine gegebenenfalls quaternisierte, aminierte Gruppe bedeutet, welche unter den folgenden Gruppen ausgewählt ist:
-N(R')-CH₂-CH₂-N(R')₂
-N(R')₂
-N⊕(R')₃A⁻
-N® (R')₂(H)A⁻
-N^{⊕}(R')(H)₂A⁻
-N(R')-CH₂-CH₂-N^{⊕}(R')(H)₂A-,
worin die Gruppen R' Wasserstoff, Phenyl, Benzyl oder eine einwertige gesättigte Kohlenwasserstoffgruppe, beispielsweise eine Alkylgruppe mit 1 bis 20 Kohlenstoffatomen, bedeuten können und A⁻ ein Halogenid ist, wie beispielsweise Fluorid, Chlorid, Bromid oder Iodid;
c) den kationischen siliconierten Polymeren der folgenden Formel: 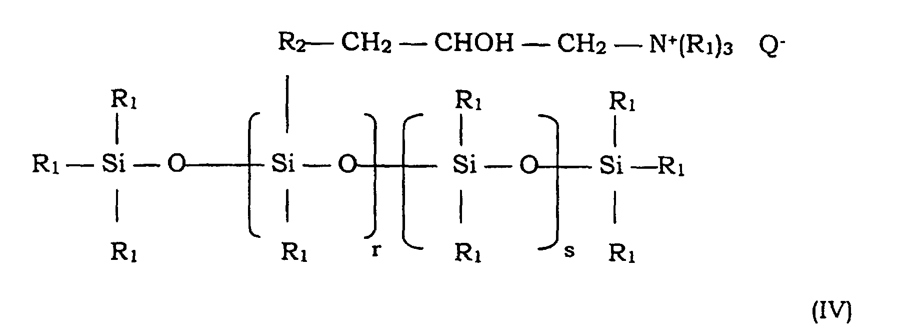 worin bedeuten:
- R₁ eine einwertige Kohlenwasserstoffgruppe mit 1 bis 18 Kohlenstoffatomen und insbesondere eine C₁₋₁₈-Alkylgruppe oder eine C₂₋₁₈-Alkenylgruppe, beispielsweise Methyl,
- R₂ eine zweiwertige Kohlenwasserstoffgruppe und insbesondere eine Alkylengruppe mit 1 bis 18 Kohlenstoffatomen oder eine zweiwertige Alkylenoxygruppe mit 1 bis 18 Kohlenstoffatomen, beispielsweise mit 1 bis 8 Kohlenstoffatomen,
- Q⁻ ein Halogenid, insbesondere Chlorid,
- r einen statistischen Mittelwert von 2 bis 20 und insbesondere von 2 bis 8, und
- s einen statistischen Mittelwert von 20 bis 200 und insbesondere von 20 bis 50.

9. Zusammensetzung nach einem der Ansprüche 1 bis 8, **dadurch gekennzeichnet, dass** die Konzentration des aminierten Silicons im Bereich von 0,05 bis 10 Gew.-%, bezogen auf das Gesamtgewicht der Zusammensetzung, und vorzugsweise im Bereich von 0,1 bis 5 % liegt.

10. Zusammensetzung nach einem der Ansprüche 1 bis 9, die gebrauchsfertig ist, zum oxidativen Färben von Keratinfasern und insbesondere menschlichen Keratinfasern, wie dem Haar, die in einem für Keratinfasern geeigneten Medium ferner mindestens eine Oxidationsbase und gegebenenfalls einen oder mehrere Kuppler enthält.

11. Zusammensetzung nach Anspruch 10, **dadurch gekennzeichnet, dass** die Oxidationsbasen unter den p-Phenylendiaminen, Doppelbasen, o- oder p-Aminophenolen und den heterocyclischen Oxidationsbasen sowie den Additionssalzen dieser Verbindungen mit einer Säure ausgewählt sind.

12. Zusammensetzung nach Anspruch 10 oder 11, **dadurch gekennzeichnet, dass** die Oxidationsbasen in Konzentrationen von 0,0005 bis 12 Gew.-% des Gesamtgewichts der Zusammensetzung vorliegen.

13. Zusammensetzung nach Anspruch 10, **dadurch gekennzeichnet, dass** die Kuppler unter den m-Phenylendiaminen, m-Aminophenolen, m-Dihydroxybenzolen, heterocyclischen Kupplern sowie den Additionssalzen dieser Verbindungen mit einer Säure ausgewählt sind.

14. Zusammensetzung nach einem der Ansprüche 10 oder 13, **dadurch gekennzeichnet, dass** die Kuppler in Konzentrationen von 0,0001 bis 10 Gew.-% des Gesamtgewichts der Zusammensetzung, vorliegen.

15. Zusammensetzung nach einem der Ansprüche 11 bis 13, **dadurch gekennzeichnet, dass** die Additionssalze der Oxidationsbasen und Kuppler mit einer Säure unter den Hydrochloriden, Hydrobromiden, Sulfaten, Tartraten, Lactaten und Acetaten ausgewählt sind.

16. Zusammensetzung nach einem der Ansprüche 10 bis 15, **dadurch gekennzeichnet, dass** sie ferner Direktfarbstoffe enthält.

17. Zusammensetzung nach einem der Ansprüche 1 bis 16, **dadurch gekennzeichnet, dass** das für Keratinfasern geeignete Medium (oder der Träger) aus Wasser oder einem Gemisch von Wasser und mindestens einem organischen Lösungsmittel besteht.

18. Zusammensetzung nach Anspruch 17, **dadurch gekennzeichnet, dass** die organischen Lösungsmittel in Mengenanteilen vorzugsweise von 1 bis 40 Gew.-%, bezogen auf das Gesamtgewicht der Zusammensetzung, und noch bevorzugter 5 bis 30 Gew.-% vorliegen können.

19. Zusammensetzung nach einem der Ansprüche 1 bis 18, **dadurch gekennzeichnet, dass** sie einen pH-Wert im Bereich von 5 bis 11 und vorzugsweise 6,5 bis 10 aufweist.

20. Zusammensetzung nach einem der Ansprüche 1 bis 19, **dadurch gekennzeichnet, dass** sie ferner mindestens einen kosmetischen Zusatzstoff enthält, der herkömmlich in Zusammensetzungen zum Färben, permanenten Verformen oder Entfärben der Haare verwendet wird und der unter den anionischen, kationischen, nichtionischen, amphoteren oder zwitterionischen grenzflächenaktiven Stoffen oder deren Gemischen, anionischen, kationischen, nichtionischen, amphoteren oder zwitterionischen Polymeren oder deren Gemischen, anorganischen oder organischen Verdickungsmitteln, Antioxidantien, Enzymen, die von den Oxidoreduktasen (2 Elektronen) verschieden sind, Penetrationsmitteln, Maskierungsmitteln, Parfums, Puffern, Dispergiermitteln, Konditioniermitteln, Filmbildnern, Konservierungsmitteln und Trübungsmitteln ausgewählt ist.

21. Verfahren zum Färben von Keratinfasern und insbesondere menschlichen Keratinfasern, wie dem Haar, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der Ansprüche 10 bis 20 aufgebracht wird.

22. Verfahren nach Anspruch 21, **dadurch gekennzeichnet, dass** es einen vorbereitenden Schritt umfasst, der darin besteht, einerseits eine Zusammensetzung (A), die in einem zum Färben geeigneten Medium mindestens eine Oxidationsbase und gegebenenfalls mindestens einen Kuppler nach einem der vorhergehenden Ansprüche enthält, und andererseits eine Zusammensetzung (B) getrennt voneinander aufzubewahren, die in einem für Keratinfasern geeigneten Medium mindestens ein Enzym vom Typ der Oxidoreduktasen (2 Elektronen) in Gegenwart mindestens eines Donors für das Enzym nach einem der vorhergehenden Ansprüche enthält, und diese bei der Anwendung zu vermischen, bevor das Gemisch auf die Fasern aufgebracht wird, wobei entweder die Zusammensetzung (A) der die Zusammensetzung (B) das in einem der vorhergehenden Ansprüche definierte aminierte Silicon enthält.

23. Vorrichtung mit mehreren Abteilungen oder "Kit" zum Färben, **dadurch gekennzeichnet, dass** sie eine erste Abteilung mit der in Anspruch 22 definierten Zusammensetzung (A) und eine zweite Abteilung mit der in Anspruch 22 definierten Zusammensetzung (B) enthält.

24. Verfahren zur Behandlung von Keratinfasern und insbesondere des Haares, um eine dauerhafte Verformung des Haares, insbesondere in Form von dauergewelltem Haar, zu erzielen, wobei das Verfahren die folgenden Schritte umfasst: (i) auf die zu behandelnden Keratinfasern wird eine reduzierende Zusammensetzung aufgebracht, wobei das keratinische Material vor, während oder nach dem Aufbringen unter mechanische Spannung gesetzt wird, (ii) das keratinische Material wird gegebenenfalls gespült, (iii) auf das gegebenenfalls gespülte keratinische Material wird eine Zusammensetzung nach einem der Ansprüche 1 bis 9 und 17 bis 20 aufgebracht und (iv) das keratinische Material wird gegebenenfalls nochmals gespült.

25. Verfahren zur Behandlung von Keratinfasern und insbesondere des Haares, um das Haares zu entfärben, wobei eine oxidierende Zusammensetzung nach einem der Ansprüche 1 bis 9 und 17 bis 20 auf die Keratinfasern aufgebracht wird, die gegebenenfalls ein zusätzliches Oxidationsmittel enthält, und in einem zweiten Schritt die Keratinfasern gespült werden.

## Claims

1. Cosmetic and/or dermatological composition intended for treating keratin fibres, in particular human keratin fibres and more particularly human hair, comprising, in a support which is suitable for keratin fibres:
(a) at least one enzyme of 2-electron oxidoreductase type in the presence of at least one donor for the said enzyme,
(b) at least one aminosilicone.

2. Composition according to Claim 1, **characterized in that** the 2-electron oxidoreductase is chosen from uricases of animal, microbiological or biotechnological origin.

3. Composition according to Claim 1 or 2, **characterized in that** the 2-electron oxidoreductase(s) represent(s) from 0.01 to 20% by weight relative to the total weight of the composition.

4. Composition according to Claim 3, **characterized in that** the 2-electron oxidoreductase(s) represent(s) from 0.1 to 5% by weight relative to the total weight of the composition.

5. Composition according to Claim 2, **characterized in that** the donor (or substrate) for the said 2-electron oxidoreductase is chosen from uric acid and its salts.

6. Composition according to any one of the preceding claims, **characterized in that** the donor(s) represent(s) from 0.01 to 20% by weight relative to the total weight of the composition.

7. Composition according to Claim 6, **characterized in that** the donor(s) represent(s) from 0.1 to 5% by weight relative to the total weight of the composition.

8. Composition according to any one of Claims 1 to 7, **characterized in that** the aminosilicone is chosen from:
**(a)** the polysiloxanes corresponding to the formula: 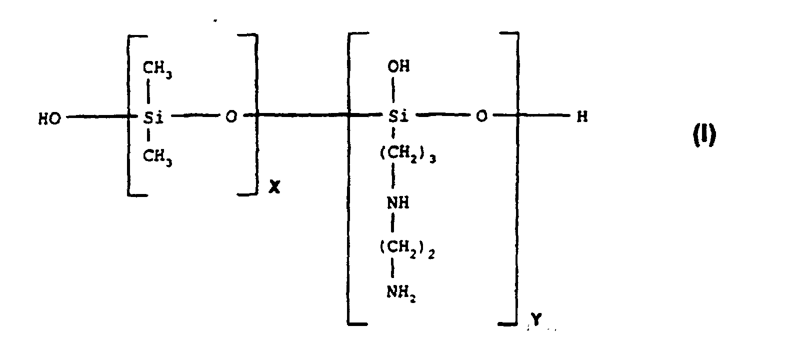 in which x and y are integers dependent on the molecular weight, generally such that the said number-average molecular weight is between 5000 and 500,000 approximately;
**(b)** cationic silicone polymers corresponding to the formula:
RₐG₃-ₐ-Si (OSiG₂)ₙ- (OSiG_{b}R_{2-b}) ₘ-O-SiG₃₋ₐ-Rₐ **(II)** (II)
in which:
G is a hydrogen atom or a phenyl, OH or C₁-C₈ alkyl, for example methyl, group,
a denotes the number 0 or an integer from 1 to 3, in particular 0,
b denotes 0 or 1, and in particular 1,
m and n are numbers such that the sum (n + m) can range especially from 1 to 2000 and in particular from 50 to 150, it being possible for n to denote a number from 0 to 1999 and in particular from 49 to 149, and it being possible for m to denote a number from 1 to 2000 and in particular from 1 to 10;
R is a monovalent radical of formula -C_{q}H_{2q}L in which q is a number from 2 to 8 and L is an optionally quaternized amine group chosen from the groups:
-N(R')-CH₂-CH₂-N(R')₂
-N(R')₂
-N⊕(R')₃A⁻
-N⊕(R')₂(H)A⁻
-N⊕(R')(H)₂A⁻
-N(R') -CH₂-CH₂-N⊕(R') (H)₂A⁻ ;
in which R' can denote hydrogen, phenyl, benzyl or a monovalent, saturated hydrocarbon-based radical, for example an alkyl radical containing from 1 to 20 carbon atoms, and A⁻ represents a halide ion such as, for example, fluoride, chloride, bromide or iodide.
**(c)** cationic silicone polymers corresponding to the formula: 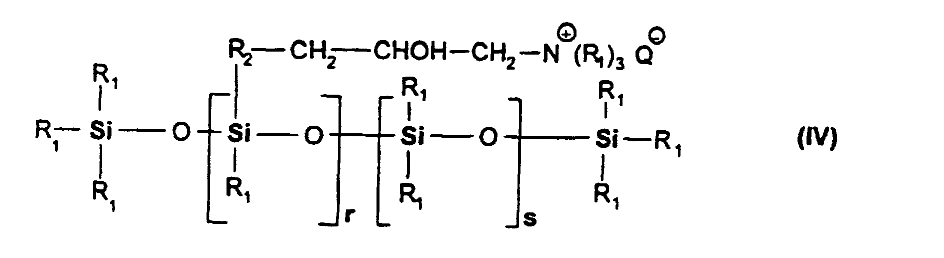 in which
R₁ represents a monovalent hydrocarbon-based radical containing from 1 to 18 carbon atoms, and in particular a C₁-C₁₈ alkyl radical or a C₂-C₁₈ alkenyl radical, for example methyl;
R₂ represents a divalent hydrocarbon-based radical, in particular a C₁-C₁₈ alkylene radical or a C₁-C₁₈, for example C₁-C₈, divalent alkyleneoxy radical;
Q⁻ is a halide ion, in particular chloride;
r represents an average statistical value from 2 to 20 and in particular from 2 to 8;
s represents an average statistical value from 20 to 200 and in particular from 20 to 50.

9. Composition according to any one of Claims 1 to 8, **characterized in that** the concentration of aminosilicone ranges from 0.05% to 10% by weight relative to the total weight of the composition, and preferably between 0.1 and 5%.

10. Ready-to-use composition according to any one of Claims 1 to 9, for the oxidation dyeing of keratin fibres, and in particular human keratin fibres such as the hair, of the type also comprising, in a medium which is suitable for keratin fibres, at least one oxidation base and, optionally, one or more couplers.

11. Composition according to Claim 10, **characterized in that** the oxidation bases are chosen from para-phenylenediamines, double bases, ortho- or para-aminophenols and heterocyclic bases, as well as the addition salts of these compounds with an acid.

12. Composition according to Claim 10 or 11, **characterized in that** the oxidation bases are present in concentrations ranging from 0.0005 to 12% by weight relative to the total weight of the composition.

13. Composition according to Claim 10, **characterized in that** the couplers are chosen from meta-phenylenediamines, meta-aminophenols, metadiphenols and heterocyclic couplers, and the addition salts of these compounds with an acid.

14. Composition according to Claim 10 or 13, **characterized in that** the couplers are present in concentrations ranging from 0.0001 to 10% by weight relative to the total weight of the composition.

15. Composition according to Claim 11 or 13, **characterized in that** the addition salts with an acid for the oxidation bases and the couplers are chosen from the hydrochlorides, hydrobromides, sulphates, tartrates, lactates and acetates.

16. Composition according to any one of Claims 10 to 15, **characterized in that** it also contains direct dyes.

17. Composition according to any one of Claims 1 to 16, **characterized in that** the medium which is suitable for the keratin fibres (or support) consists of water or of a mixture of water and at least one organic solvent.

18. Composition according to Claim 17, **characterized in that** the organic solvents can be present in proportions preferably ranging from 1 to 40% by weight relative to the total weight of the composition, and even more preferably ranging from 5 to 30% by weight.

19. Composition according to any one of Claims 1 to 18, **characterized in that** the pH ranges from 5 to 11 and preferably from 6.5 to 10.

20. Composition according to any one of Claims 1 to 19, **characterized in that** it also contains at least one cosmetic adjuvant used conventionally in compositions for dyeing, permanently reshaping or bleaching the hair, chosen from the group consisting of anionic, cationic, nonionic, amphoteric or zwitterionic surfactants or mixtures thereof, anionic, cationic, nonionic, amphoteric or zwitterionic polymers or mixtures thereof, inorganic or organic thickeners, antioxidants, enzymes other than the 2-electron oxidoreductases, penetration agents, sequestering agents, fragrances, buffers, dispersing agents, conditioners, film-forming agents, preserving agents and opacifiers.

21. Process for dyeing keratin fibres, and in particular human keratin fibres such as the hair, **characterized in that** at least one ready-to-use dye composition as defined in any one of Claims 10 to 20 is applied to the said fibres, for a period which is sufficient to develop the desired coloration.

22. Process according to Claim 21, **characterized in that** it includes a first step which consists in separately storing, on the one hand, a composition (A) comprising, in a medium which is suitable for dyeing, at least one oxidation base and optionally at least one coupler as defined in any one of the preceding claims, and, on the other hand, a composition (B) containing, in a medium which is suitable for keratin fibres, at least one enzyme of 2-electron oxidoreductase type in the presence of at least one donor for the said enzyme as defined in any one of the preceding claims, and then in mixing them together at the time of use, before applying this mixture to the keratin fibres; composition (A) or composition (B) containing the aminosilicone as defined in the preceding claims.

23. Multi-compartment dyeing device or kit, **characterized in that** it contains a first compartment containing composition (A) as defined in Claim 22 and a second compartment containing composition (B) as defined in Claim 22.

24. Process for treating keratin fibres, in particular the hair, in order to obtain a permanent reshaping of this hair, in particular in the form of permanent-waved hair, this process comprising the following steps: (i) a reducing composition is applied to the keratin fibres to be treated, the keratin substance being placed under mechanical tension before, during or after the said application, (ii) the keratin substance is optionally rinsed, (iii) an oxidizing composition as defined in any one of Claims 1 to 9 and 17 to 20 is applied to the optionally rinsed keratin substance, (iv) the keratin substance is optionally rinsed again.

25. Process for treating keratin fibres, in particular the hair, in order to bleach them, this process comprising the application of an oxidizing composition as defined in any one of Claims 1 to 9 and 17 to 20 optionally containing an auxiliary oxidizing agent and a second step of rinsing the keratin fibres.
